# FASCICULE DE BREVET EUROPEEN

(11) **EP 0 912 718 B1**
(45) Date de publication et mention de la délivrance du brevet: **20.09.2006**
(21) Numéro de dépôt: 97930583.6
(22) Date de dépôt: 25.06.1997
(51) Int. Cl.: C12H 1/00, C12H 1/14, C12P 21/00

(54) **PRODUIT DE STABILISATION PROTEIQUE DES VINS**
PRODUKT ZUR PROTEINSTABILISIERUNG VON WEINEN
METHOD FOR PROTEIN STABILISATION OF WINES

(30) Priorité: 26.06.1996 FR 9608187
(43) Date de publication de la demande: 06.05.1999
(73) Titulaire: Societe d'Applications de Recherches et de Conseils Oenologiques (SARCO), 33000 Bordeaux (FR)
(72) Inventeur: MOINE, Virginie, F-33600 Pessac (FR); DUBOURDIEU, Denis, F-33410 Beguey (FR)
(74) Mandataire: Thébault, Jean-Louis
(86) Numéro de dépôt international: PCT/FR1997/001130
(87) Numéro de publication internationale: WO 1997/049794

(56) Documents cités:
- EP-A- 0 582 244
- FR-A- 2 726 284
- DATABASE WPI Section Ch, Week 9626 Derwent Publications Ltd., London, GB; Class B04, AN 96-253874 XP002027737 & JP 08 103 271 A (ORIENTAL YEAST CO LTD) , 23 avril 1996
- DATABASE WPI Section Ch, Week 7406 Derwent Publications Ltd., London, GB; Class D16, AN 74-10288V XP002027749 & JP 49 001 870 B (AMANO SEIYAKU K K) , 17 janvier 1974

## Description

La présente invention a pour objet des procédés concernant la stabilisation protéique des vins.

Dans la demande de brevet N° 2 726 284 au nom de la Faculté d'Oenologie de Bordeaux, il a été décrit et revendiqué des mannoprotéines extraites par voie enzymatique dont l'action pour lutter contre les précipitations de sels tartriques et protéiques est particulièrement efficace.

Plus particulièrement, ces mannoprotéines étaient obtenues par digestion enzymatique de parois de levures, sous l'action des β 1-3 et β 1-6 glucanases, lesdites levures étant dans un mode de réalisation, l'espèce *Saccharomyces cerevisiae.*

On avait pu constater aussi que la mannoprotéine de masse moléculaire 31 800 Daltons, nommée MP 32, était plus spécifiquement efficace, vis à vis de la stabilisation protéique.

Aussi des essais ont été menés pour montrer que MP32 est un fragment d'invertase.

L'invention concerne donc un procédé d'obtention de mannoprotéines par voie enzymatique à partir d'invertase en vue de la stabilisation protéique des vins blancs qui consiste à réaliser une digestion enzymatique d'invertase de *Saccharomyces cerevisiae* par hydrolyse.

Cette digestion enzymatique est réalisée en présence de β 1-3 et β 1-6 glucanase et de protéases actives en milieux acides.

L'hydrolysat récupéré subit un traitement physique comprenant notamment une dialyse contre de l'eau sur membrane avec un seuil de coupure de 6 000 à 8 000 Daltons, suivie d'une lyophilisation avec une centrifugation et une filtration préalablement à la dialyse. S. cerevisiae

L'invention concerne aussi le choix des écorces de levure/susceptibles de présenter une activité invertasique en sorte de générer des mannoprotéines de masse moléculaire adaptée pour stabiliser les vins blancs comme définie dans la revendication 5.

Le procédé fait l'objet d'essais qui sont décrits ci-après, en regard des figures qui représentent :
- figure 1, un diagramme comparatif de l'effet de stabilisation obtenu, par la quantité de bentonite nécessaire pour la stabilisation du vin et par l'indice de turbidité NTU, reflétant la densité du trouble formé après le test à la chaleur,
- figure 2, diagramme comparatif de la teneur en MP32 des différentes préparations, réalisé par électrophorèse capillaire,
- figure 3, un diagramme comparatif de l'effet de stabilisation obtenu avec les différentes préparations en fonction des types d'hydrolysats d'invertase, et
- figure 4, la teneur comparée en MP32 des différentes préparations utilisées pour obtenir les résultats de la figure 3.

On sait que l'invertase 2 ou 4 de *Saccharomyces cerevisiae* a une masse moléculaire de 270 000 Daltons ce qui est beaucoup plus important que la masse moléculaire de la MP32.

On sait aussi que l'invertase comprend notamment l'enchaînement suivant d'acides aminés :

Lysine-Valine-Phenylalanine-Tryptophane-Tyrosine-Glutamine-Proline-Sérine-Glutagine-Lysine.

De façon surprenante, le demandeur a réalisé un séquençage de la mannoprotéine MP32 et a constaté 100% d'homologie avec la partie de séquence contenue dans l'invertase.

Afin de vérifier que la mannoprotéine MP32 est bien issue d'un fragment d'invertase de *Saccharomyces cerevisiae*, il est proposé de réaliser les deux préparations suivantes :
- Inv1, 30 à 50 unités/mg d'invertase commerciale insoluble SIGMA ® , hydrolysées dans 5% de Glucanex® , de la firme NOVO, et
- Inv2, 400 unités/mg d'invertase commerciale soluble SIGMA® , hydrolysées dans 5% de Glucanex® , de la firme NOVO.

Après digestion, les hydrolysats Inv1g et Inv2g sont centrifugés, filtrés et dialysés avec une membrane ayant un seuil de coupure de l'ordre de 6 000 à 8000 Daltons, l'hydrolysat ainsi obtenu est lyophylisé.

On additionne ensuite 25 g/hl de chacune des préparations à un vin en vue de réaliser un état comparatif de l'effet de stabilisation de chacune de ces préparations.

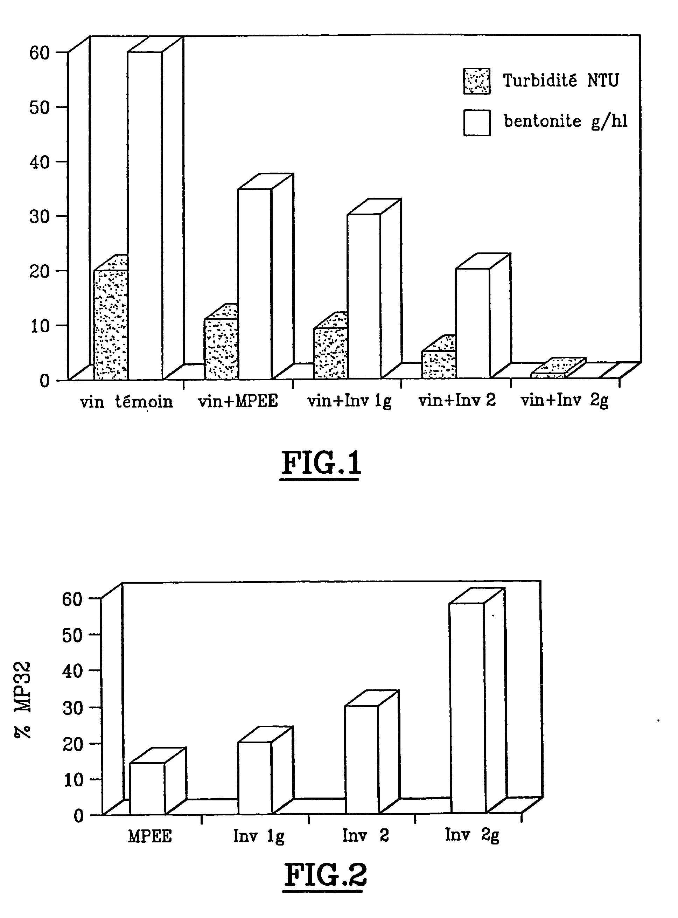

Les résultats sont regroupés sur le diagramme de la figure 1, chaque vin ayant subi un traitement à la chaleur, 80 °C pendant 30 minutes afin d'estimer la stabilité protéique.

On constate que la mannoprotéine extraite par voie enzymatique à partir des parois de levure digérées avec du Glucanex a un effet stabilisant puisqu'il faut moins de bentonite pour un indice de turbidité NTU meilleur.

A faible concentration d'invertase, la préparation d'Inv1g est encore meilleure que la préparation extraite par voie enzymatique à partir de parois de levures.

On remarque la stabilisation accrue dès que la concentration en invertase augmente, même avec la préparation non hydrolysée.

La stabilité est très grandement améliorée dès que, à forte concentration initiale d'invertase, on hydrolyse l'Inv2 par le Glucanex (Inv2g).

Ainsi, plus la préparation est riche en invertase, plus son hydrolysat par le Glucanex est actif.

Il n'est plus nécessaire d'ajouter de bentonite et la formation d'un trouble à l'issue du test à la chaleur est quasiment nulle.

Par électrophorèse capillaire, on peut constater que la teneur en MP32 évolue parallèlement avec l'activité stabilisatrice. Plus il y a de MP32, plus l'activité stabilisatrice est sensible.

On peut penser que la libération du fragment d'invertase de *Saccharomyces cerevisiae* au cours de l'autolyse des levures, contenant l'enchaînement adapté d'acides aminés est due à l'action de protéases.

Compte tenu des paramètres physico-chimiques des vins, pH 3 à 3,8, les seules protéases de *Saccharomyces cerevisiae* susceptibles d'être actives sont :
- la protéase A, SIGMA® et
- la carboxypeptidase Y, SIGMA® .

On réalise les essais suivants :

Dans une solution tampon citrate de 10 ml, on hydrolyse 100 mg d'invertase purifiée Inv2, pendant 15 heures à 20°C, avec :
- 5 mg de Glucanex,
- 2,5 mg de carboxypeptidase Y,
- 0,3 mg de protéase A.

En parallèle, on réalise une hydrolyse similaire mais en inactivant ces mêmes enzymes par la chaleur (1 mn à 100°C).

Ensuite, on dialyse contre de l'eau les différentes préparations avec une membrane ayant comme précédemment un seuil de coupure de 6 000 à 8 000 Daltons, puis elles sont lyophilisées.

De façon identique, des échantillons de vin reçoivent à raison de 25 g/hl les préparations puis les vins subissent un test à la chaleur en sorte de contrôler l'indice de turbidité.

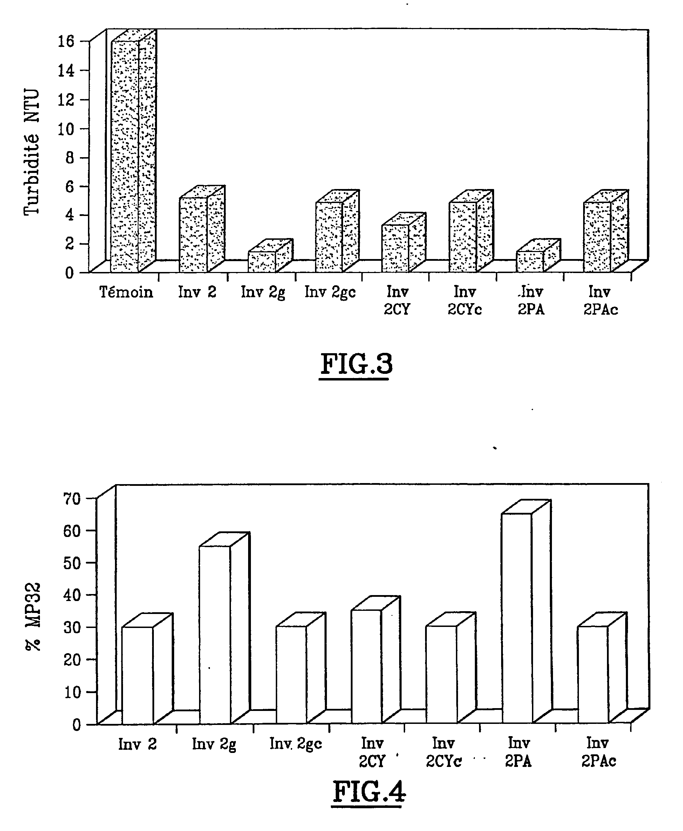

Dans le diagramme de la figure 3, les abréviations correspondent au produits suivants :
- *g*, Glucanex
- *gc*,Glucanex, mais inactivation préalable à 100°C pendant une minute,
- *CY*, carboxypeptidase Y,
- *Cyc*, carboxypeptidase Y, mais inactivation préalable à 100°C pendant une minute,
- *PA*, protéase A, et
- *PAc*, protéase A, mais inactivation préalable à 100°C pendant une minute.

On constate que l'hydrolyse enzymatique d'une préparation d'invertase conduit à des produits ayant une action stabilisatrice sur le vin blanc.

On remarque l'action particulièrement efficace du Glucanex et de la protéase A pour générer des mannoprotéines MP32. La protéase A est encore plus efficace.

Ceci est d'ailleurs confirmé par les essais récapitulés sur la figure 4 qui montre la teneur en MP32 des différents hydrolysats précédents.

On note aussi que la désactivation partielle par la chaleur a un effet négatif sur les résultats obtenus.

On a ainsi montré que le procédé procure une action stabilisatrice des protéines des vins blancs.

## Revendications

1. Procédé de fabrication d'un produit biologique ayant un effet de stabilisation des précipitations protéiques des vins blancs, **caractérisé en ce qu'**il consiste à réaliser une digestion enzymatique d'invertase de *Saccharomyces cerevisiae* par hydrolyse, en présence de β1-3 et β1-6 glucanase, et de protéases actives en milieux acide.

2. Procédé selon la revendication 1, **caractérisé en ce que** l'hydrolysat récupéré subit un traitement physique.

3. Procédé selon la revendication 2, **caractérisé en ce que** le traitement physique comprend une dialyse contre de l'eau sur membrane avec un seuil de coupure de 6 000 à 8 000 Daltons, suivi d'une lyophilisation.

4. Procédé selon la revendication 2 ou 3, **caractérisé en ce que** le traitement physique comprend en outre une centrifugation et une filtration préalablement à la dialyse.

5. Procédé de choix de levures *Saccharomyces cerevisiae* pouvant générer, à partir de leurs écorces des mannoprotéines de masse moléculaire adaptée en vue de la stabilisation des vins blancs, **caractérisé en ce que** les levures écorces sont choisies parmi les levures dont les écorces présentent une activité invertasique.

## Claims

1. A process for the production of a biological product having a stabilizing effect on protein precipitations in white wines, **characterized in that** the process consists of carrying out an enzymatic digestion of invertase of *Saccharomyces cerevisiae* by hydrolysis in the presence of β1-3 glucanase and β1-6 glucanase, and active proteases in acid media.

2. The process according to claim 1, **characterized in that** the hydrolysate recovered undergoes a physical treatment.

3. The process according to claim 2, **characterized in that** the physical treatment comprises a dialysis against water across a membrane with a cutoff threshold of 6,000 to 8,000 Daltons, followed by lyophilization.

4. The process according to claim 2 or 3, **characterized in that** the physical treatment furthermore comprises centrifugation and filtration prior to dialysis.

5. The process of choosing *Saccharomyces cerevisiae* yeasts that may generate, from their mannoprotein cortexes, a molecular mass adapted for stabilizing white wines, **characterized in that** the yeast cortexes are chosen from among yeasts whose cortexes present an invertase activity.

## Patentansprüche

1. Verfahren zur Herstellung eines biologischen Produkts, welches eine stabilisierende Wirkung von Proteinausfallprodukten aus Weißweinen aufweist, **dadurch gekennzeichnet, dass** es in der Umsetzung eines enzymatischen Invertaseverdaus von *Saccharomyces cerevisiae* durch Hydrolyse in Gegenwart von β1-3 und β1-6 Glucanase und von in saurer Umgebung aktiven Proteasen besteht.

2. Verfahren nach Anspruch 1, **dadurch gekennzeichnet, dass** das erhaltene Hydrolysat einer physikalischen Behandlung unterzogen wird.

3. Verfahren nach Anspruch 2, **dadurch gekennzeichnet, dass** die physikalische Behandlung eine Membrandialyse gegen Wasser mit einer Ausschlussgrenze von 6000 bis 8000 Dalton, gefolgt von einer Gefriertrocknung, umfasst.

4. Verfahren nach Anspruch 2 oder 3, **dadurch gekennzeichnet, dass** die physikalische Behandlung ferner eine Zentrifugation und eine Filtration vor der Dialyse umfasst.

5. Verfahren zur Auswahl von *Saccharomyces cerevisiae* Hefen, welche ausgehend von ihren Zellwänden Mannoproteine einer Molekularmasse erzeugen können, die hinsichtlich der Stabilisation von Weißweinen angepasst ist, **dadurch gekennzeichnet, dass** die Hefezellwände ausgewählt sind aus Hefen, deren Zellwände eine Invertaseaktivität zeigen.
